# EUROPEAN PATENT APPLICATION

(11) **EP 0 575 923 A1**
(43) Date of publication of application: **29.12.1993**
(21) Application number: 93109829.7
(22) Date of filing: 19.06.1993
(51) Int. Cl.: C07D 403/06, C07D 207/32, A61K 31/41

(54) **Triazole Derivatives**

(30) Priority: 24.06.1992 JP 165777/92
(71) Applicant: Ikeda Mohando Co., Ltd., Nakaniikawa-gun Toyama-ken (JP)
(72) Inventor: Ogawa, Kazu, Toyama-shi, Toyama-ken (JP)
(74) Representative: Werner, Hans-Karsten, Dr.Dipl.-Chem.

(57) **Abstract**

A triazole derivative having antifungal activity is disclosed. The compounds are represented by the following formula: 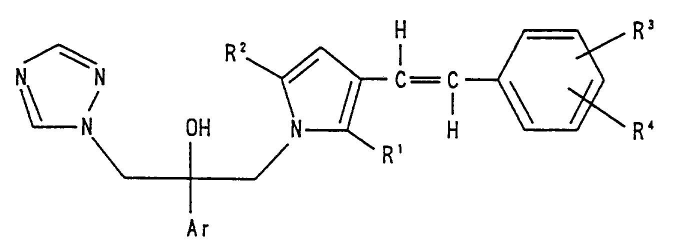
wherein Ar represents a phenyl group or a phenyl group substituted with one or two groups selected from the group consisting of halogen atoms and trifluoromethyl group; R¹ and R² independently represent a hydrogen atom, halogen atom, or a lower alkyl group; R³ and R⁴ independently represent a hydrogen atom, halogen atom, nitro group, a lower alkyl group, a lower halogeno-alkyl group, a lower alkoxy group, a lower halogeno-alkoxy group, non-substituted or substituted aryl group, non-substituted or substituted amino group, or a lower alkylthio group.

## Description

### BACKGROUND OF THE INVENTION

### Field of the Invention

The present invention relates to triazole derivatives and pharmacologically acceptable salts thereof having excellent antifungal activity and are useful as antifungal agent.

The present invention also relates to synthetic intermediates useful for the preparation of said triazole derivatives.

### Description of Related Art

Amphotericin B and Flucytosine have been clinically used as medicament for the treatment of mycosis profunda. Various triazole type compounds are reported as useful antifungal agents (see, for instance, Japanese Patent Unexamined Publication Nos. (Sho)59-175475/1984 and (Sho)61-72767/1986 in which compounds of the following Formula are disclosed). 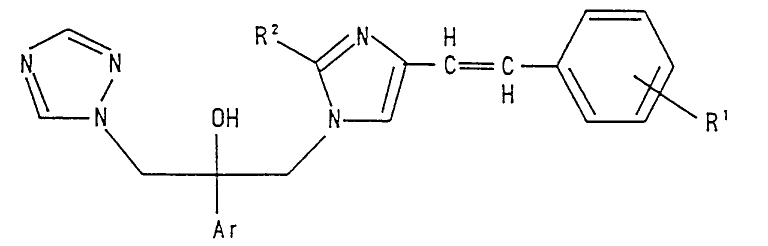
Fluconazole (trade name: Diflucan), one of triazole type antifungal agents, has recently been used clinically. However, these antifungal agents are not fully satisfactory as medicaments for the treatment of mycosis profunda from a standpoit of bioavailability or toxicity. Accordingly, an orally available medicament for the treatment of mycosis profunda have been desired.

### SUMMARY OF THE INVENTION

An object of the present invention is to provide compounds having excellent antifungal activity.

Another object of the present invention is to provide a pharmaceutical composition comprising an effective amount of said compound which is useful as an orally available medicament for the treatment of mycosis profunda.

The inventor conducted various studies to achive the foregoing object, and as a results, found that triazole derivatives represented by formula (1) have excellent antifungal activities, and that a pharmaceutical composition comprising said compound is useful as an orally available medicament for the treatment of mycosis profunda. The present invention was achieved on the basis of these findings.

The present invention thus provide triazole derivatives represented by the following formula (1) and pharmacologically acceptable salts thereof: 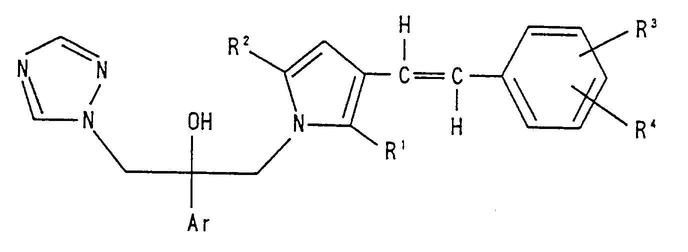
wherein Ar represents a phenyl group or a phenyl group substituted with one or two substituents selected from the group consisting of halogen atoms and trifluoromethyl group; R¹ and R² independently represent a hydrogen atom, halogen atom, or a lower alkyl group; R³ and R⁴ independently represent a hydrogen atom, halogen atom, nitro group, a lower alkyl group, a lower halogeno-alkyl group, a lower alkoxy group, a lower halogeno-alkoxy group, non-substituted or substituted aryl group, non-substituted or substituted amino group, or a lower alkylthio group.

The present invention also provides a pharmaceutical composition useful for the treatment of mycosis profunda which comprises an effective amount of the above-described compound of formula (1) together with a pharmaceutically acceptable carrier or coating.

### DETAILED EXPLANATION OF THE PREFERRED EMBODIMENT

Where Ar is a phenyl group substituted with one or two halogen atoms, the halogen atom may be fluorine atom, chlorine atom, bromine atom, or iodine atom. Where the phenyl group is substituted with two halogen atoms, the halogen atoms may be the same or different atoms which may be selected from fluorine atom, chlorine atom, bromine atom, or iodine atom. Examples of the phenyl groups substituted with one or two halogen atoms include, for example, 2-trifluoromethylphenyl group, 3-trifluoromethylphenyl group, 4-trifluoromethylphenyl group, 2-fluorophenyl group, 3-fluorophenyl group, 4-fluorophenyl group, 2-chlorophenyl group, 3-chlorophenyl group, 4-chlorophenyl group, 2-chloro-4-fluorophenyl group, 2-fluoro-4-chlorophenyl group, 4-bromophenyl group, 2-fluoro-4-iodophenyl group, 2,4-dichlorophenyl group, 2,4-difluorophenyl group, 3,4-difluorophenyl group, 2-fluoro-4-trifluoromethylphenyl group, 2-trifluoromethyl-4-chlorophenyl group, and 2-trifluoromethyl-4-fluorophenyl group.

R¹ and R² independently represents a hydrogen atom, halogen atom, or a lower alkyl group. The halogen atom may be fluorine atom, chlorine atom, bromine atom, or iodine atom. The lower alkyl group may be a straight- or blanched-chain alkyl group having 1 to 6 carbon atoms. Examples of the lower alkyl groups include, for example, methyl group, ethyl group, n-propyl group, isopropyl group, n-butyl group, isobutyl group, s-butyl group, t-butyl group, n-pentyl group, and n-hexyl group.

R³ and R⁴ independently represent a hydrogen atom, halogen atom, nitro group, a lower alkyl group, a lower halogeno-alkyl group, a lower alkoxy group, a lower halogeno-alkoxy group, non-substituted or substituted aryl group, non-substituted or substituted amino group, or a lower alkylthio group. The halogen atom may be fluorine atom, chlorine atom, bromine atom, or iodine atom. The lower alkyl group may be a straight- or blanched-chain alkyl group having 1 to 6 carbon atoms. Examples of the lower alkyl groups include, for example, methyl group, ethyl group, n-propyl group, isopropyl group, n-butyl group, isobutyl group, s-butyl group, t-butyl group, n-pentyl group, and n-hexyl group. The lower halogeno-alkyl group may be a straight- or blanched-chain alkyl group having 1 to 6 carbon atoms which is substituted with one or more halogen atoms. The halogen atom may be fluorine atom, chlorine atom, bromine atom, or iodine atom. For example, fluoromethyl group, chloromethyl group, difluoromethyl group, dichloromethyl group, trifluoromethyl group, and trichloromethyl group may be used.

Where R³ and R⁴ represent a lower alkoxy group, the alkoxy group may be a straight- or blanched chain alkoxy group having 1 to 6 carbon atoms. For example, methoxy group, ethoxy group, n-propoxy group, isopropoxy group, n-butoxy group, isobutoxy group, s-butoxy group, t-butoxy group, n-pentyloxy group, and n-hexyloxy group may be used. The lower halogeno-alkoxy group may be a straight- or blanched chain alkoxy group having 1 to 6 carbon atoms which is substituted with one or more halogen atoms. The halogen atom may be fluorine atom, chlorine atom, bromine atom, or iodine atom. For example, difluoromethoxy group, trifluoromethoxy group, 3,3,3,2,2-pentafluoropropoxy group, 2,2,2-trifluoroethoxy group, 2,2,3,3-tetrafluoropropoxy group, or 2,2,2-trichloroethoxy group may be used. The non-substituted aryl group may be phenyl group, naphthyl group, or indenyl group. The substituted aryl group may be a phenyl group or naphthyl group substituted with at least one halogen atom, alkyl group, alkoxy group, amino group, nitro group, hydroxy group, carboxyl group, or sulfonic acid group. The substituted amino group may be, for example, mono- or di-alkyl substituted amino groups such as methylamino group, dimethylamino group, ethylamino group, diethylamino group, n-propylamino group, n-hexylamino group, or di-n-hexylamino group; acylamino groups such as, for example, formylamino group, acetylamino group, or propionylamino group; or aralkylamino group such as benzylamino group. The lower alkylthio group may be a thiol group substituted with a straight- or branched chain alkyl group having 1 to 6 carbon atoms. Examples of the lower alkylthio groups include methylthio group, ethylthio group, n-propylthio group, isopropylthio group, pentylthio group, and n-hexylthio group.

It should be understood that salts of the compounds represented by the formula (1) fall within the scope of the present invention. Examples of pharmacologically acceptable salts include, for example, inorganic acid addition salts such as, for example, hydrochloride, hydrobromide, hydrofluoride, hydroiodide, sulfate, phosphate, or nitrate; and organic acid addition salts such as, for example, formate, acetate, propionate, succinate, maleate, fumarate, malate, citrate, salicylate, methansulfonate, benzenesulfonate, or toluenesulfonate. Hydrates or solvates as well as any forms of crystals also fall within the scope of the present invention.

Examples of the compound of the present invention include, for example,
1-[3-[2-(4-chlorophenyl)-trans-ethenyl]pyrrol-1-yl]-2-(2,4-difluorophenyl)-3-(1H-1,2,4-triazol-1-yl)propan-2-ol;
1-[3-[2-(4-nitrophenyl)-trans-ethenyl]pyrrol-1-yl]-2-(2,4-difluorophenyl)-3-(1H-1,2,4-triazol-1-yl)propan-2-ol;
1-[3-[2-(4-bromophenyl)-trans-ethenyl]pyrrol-1-yl]-2-(2,4-difluorophenyl)-3-(1H-1,2,4-triazol-1-yl)propan-2-ol;
1-[3-[2-(3-chlorophenyl)-trans-ethenyl]pyrrol-1-yl]-2-(2,4-difluorophenyl)-3-(1H-1,2,4-triazol-1-yl)propan-2-ol;
1-[3-[2-(2-chlorophenyl)-trans-ethenyl]pyrrol-1-yl]-2-(2,4-difluorophenyl)-3-(1H-1,2,4-triazol-1-yl)propan-2-ol;
1-[3-[2-(4-fluorophenyl)-trans-ethenyl]pyrrol-1-yl]-2-(2,4-difluorophenyl)-3-(1H-1,2,4-triazol-1-yl)propan-2-ol;
1-[3-[2-(2,4-dichlorophenyl)-trans-ethenyl]pyrrol-1-yl]-2-(2,4-difluorophenyl)-3-(1H-1,2,4-triazol-1-yl)propan-2-ol;
1-[3-[2-(3,4-dichlorophenyl)-trans-ethenyl]pyrrol-1-yl]-2-(2,4-difluorophenyl)-3-(1H-1,2,4-triazol-1-yl)propan-2-ol;
1-[3-[2-(2,6-dichlorophenyl)-trans-ethenyl]pyrrol-1-yl]-2-(2,4-difluorophenyl)-3-(1H-1,2,4-triazol-1-yl)propan-2-ol;
1-[3-[2-(4-trifluoromethylphenyl)-trans-ethenyl]pyrrol]-1-yl]-2-(2,4-difluorophenyl)-3-(1H-1,2,4-triazol-1-yl)propan-2-ol;
1-[3-[2-(4-methylphenyl)-trans-ethenyl]pyrrol-1-yl]-2-(2,4-difluorophenyl)-3-(1H-1,2,4-triazol-1-yl)propan-2-ol;
1-[3-[2-(4-t-butylphenyl)-trans-ethenyl]pyrrol-1-yl]-2-(2,4-difluorophenyl)-3-(1H-1,2,4-triazol-1-yl)propan-2-ol;
1-[3-[2-(4-methoxyphenyl)-trans-ethenyl]pyrrol-1-yl]-2-(2,4-difluorophenyl)-3-(1H-1,2,4-triazol-1-yl)propan-2-ol;
1-[3-[2-(4-n-hexyloxyphenyl)-trans-ethenyl]pyrrol-1-yl]-2-(2,4-difluorophenyl)-3-(1H-1,2,4-triazo-1-yl)propan-2-ol;
1-[3-[2-[4-(2,2,2-trifluoroethyl)oxyphenyl]-trans-ethenyl]pyrrol-1-yl]-2-(2,4-difluorophenyl)-3-(1H-1,2,4-triazol-1-yl)propan-2-ol;
1-[3-[2-[4-(2,2,3,3-tetrafluoropropyl)oxyphenyl]-trans-ethenyl]pyrrol-1-yl]-2-(2,4-difluorophenyl)-3-(1H-1,2,4-triazol-1-yl)propan-2-ol;
1-[3-[2-(4-biphenyl)-trans-ethenyl]pyrrol-1-yl]-2-(2,4-difluorophenyl)-3-(1H-1,2,4-triazol-1-yl)propan-2-ol;
1-[3-[2-[4-(N,N-dimethyl)aminophenyl]-trans-ethenyl]pyrrol-1-yl]-2-(2,4-difluorophenyl)-3-(1H-1,2,4-triazol-1-yl)propan-2-ol;
1-[3-[2-(4-aminophenyl)-trans-ethenyl]pyrrol-1-yl]-2-(2,4-difluorophenyl)-3-(1H-1,2,4-triazol-1-yl)propan-2-ol;
1-[3-[2-(4-acetoaminophenyl)-trans-ethenyl]pyrrol-1-yl]-2-(2,4-difluorophenyl)-3(1H-1,2,4-triazol-1-yl)propan-2-ol;
1-[3-[2-(4-methylthiophenyl)-trans-ethenyl]pyrrol-1-yl]-2-(2,4-difluorophenyl)-3-(1H-1,2,4-triazol-1-yl)propan-2-ol;
1-[3-[2-(4-chlorophenyl)-trans-ethenyl]pyrrol-1-yl]-2-(4-chlorophenyl)-3-(1H-1,2,4-triazol-1-yl)propan-2-ol;
1-[3-[2-(4-chlorophenyl)-trans-ethenyl)pyrrol-1-yl]-2-(2,4-dichlorophenyl)-3-(1H-1,2,4-triazol-1-yl)propan-2-ol;
1-[3-[2-(4-bromophenyl)-trans-ethenyl]pyrrol-1-yl]-2-(2,4-dichlorophenyl)-3-(1H-1,2,4-triazol-1-yl)propan-2-ol;
1-[3-[2-(4-chlorophenyl)-trans-ethenyl]pyrrol-1-yl]-2-(4-trifluoromethylphenyl)-3-(1H-1,2,4-triazol-1-yl)propan-2-ol;
1-[2,5-dimethyl-3-[2-(4-methylthio)phenyl]-trans-ethenyl]pyrrol-1-yl]-2-(2,4-difluorophenyl)-3-(1H-1,2,4-triazol-1-yl)propan-2-ol;
1-[2-chloro-3-[2-(4-chlorophenyl)-trans-ethenyl]pyrrol-1-yl]-2-(2,4-difluorophenyl)-3-(1H-1,2,4-triazol-1-yl)propan-2-ol;
1-[2-chloro-3-[2-(4-methylphenyl)-trans-ethenyl]pyrrol-1-yl]-2-(2,4-difluorophenyl)-3-(1H-1,2,4-triazol-1-yl)propan-2-ol;
1-[2-chloro-3-[2-[4-(2,2,3,3-tetrafluoropropyl)oxypheny]-trans-ethenyl]pyrrol-1-yl]-2-(2,4-difluorophenyl)-3-(1H-1,2,4-triazol-1-yl)propan-2-ol;
1-[2,5-dichloro-3-[2-(4-chlorophenyl)-trans-ethenyl]pyrrol-1-yl]-2-(4-trifluoromethylphenyl)-3-(1H-1,2,4-triazol-1-yl)propan-2-ol; and
1-[3-[2-(4-chlorophenyl)-trans-ethenyl]pyrrol-1-yl]-2-(2,4-difluorophenyl)-3-(1H-1,2,4-triazol-1-yl)propan-2-ol paratoluenesulfonate.

Among these compounds,
1-[3-[2-(4-chlorophenyl)-trans-ethenyl]pyrrol-1-yl]-2-(2,4-difluorophenyl)-3-(1H-1,2,4-triazol-1-yl)propan-2-ol;
1-[3-[2-(4-trifluoromethylphenyl)-trans-ethenyl]pyrrol-1-yl]-2-(2,4-difluorophenyl)-3-(1H-1,2,4-triazol-1-yl)propan-2-ol; and
1-[2-chloro-3-[2-(4-chlorophenyl)-trans-ethenyl]pyrrol-1-yl]-2-(2,4-difluorophenyl)-3-(1H-1,2,4-triazol-1-yl)propan-2-ol are preferred.
However, the compounds of the present invention are not limited to these exemplified compounds.

The compound of the present invention represented by formula (1) can be prepared according to the process described below. In the scheme, Ar and R¹ to R⁴ are the same as those defined above. 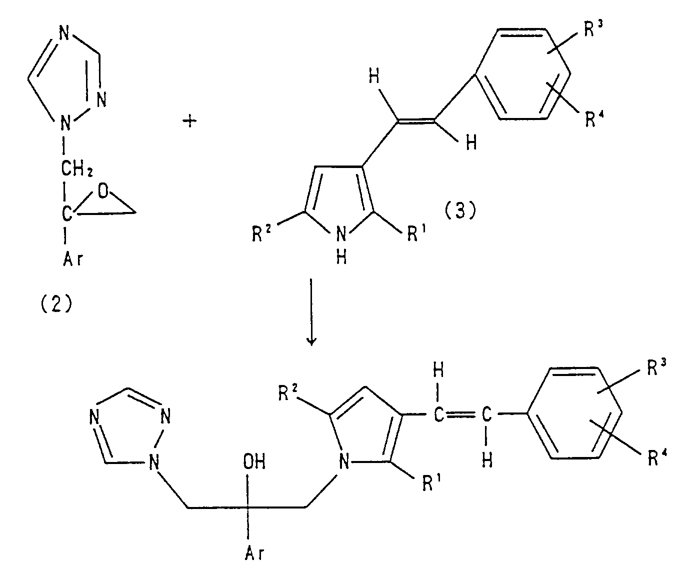
Although these reactions can be carried out in the absense of a solvent, an inert solvent may preferably used to control reaction temperature. Examples of the solvent used include, for example, N,N-dimethylformamide (DMF), N,N-dimethylacetamide, dimethyl sulfoxide (DMSO), dioxane, dimethoxyethane, tetrahydrofuran (THF), and ethylenglycol dimethylether. Among these solvents, polar solvents such as DMF or DMSO are preferably used. The reaction may be carried out at a temperature of from 20 to 200°C. Preferably, the reaction is carried out at a temperature of from 80 to 120 °C with stirring. The reaction time may usually be 30 minutes to 24 hours depending on a reaction temperature. In these reactions, a base catalyst is preferably used to accerelate the reaction rate. Examples of the base used include, for example, sodium hydride (NaH), potassium carbonate (K₂CO₃ ), sodium carbonate (Na₂CO₃ ), sodium methoxide, and sodium ethoxide. Sodium hydride is preferably used. The amount of the base may be 1 to 3 moles based on the compound of formula (3). Compounds of formula (2) used in these reactions are known to the public and can be prepared, for example, according to the method described in the Japanese Patent Unexamined Publication No. (Sho)58-32868/1983 or its analogous processes. The compound of formula (3) may be used in a ratio of 1 to 2.5 moles, preferably 1.2 moles, based on the compound of formula (2).

According to another embodiment of the present invention, there is provided a synthetic intermediate useful for preparing the triazole derivatives of the present invention represented by formula (1). The intermediate compound is represented by formula (3) shown in the aforementioned scheme, wherein R¹ and R² independently represent a hydrogen atom, a halogen atom, or a lower alkyl; R³ and R⁴ independently represent a hydrogen atom, a halogen atom, nitro group, a lower alkyl group, a lower halogeno-alkyl group, a lower alkoxy group, a lower halogeno-alkoxy group, a non-substituted or substituted aryl group, a non-substituted or substituted amino group, or a lower alkylthio group. The synthetic intermediate compound can be prepared by the process illustrated below. The preparation of compound of formula (3) will be shown in the following schemes, in which R¹ to R⁴ are the same as those defined above. 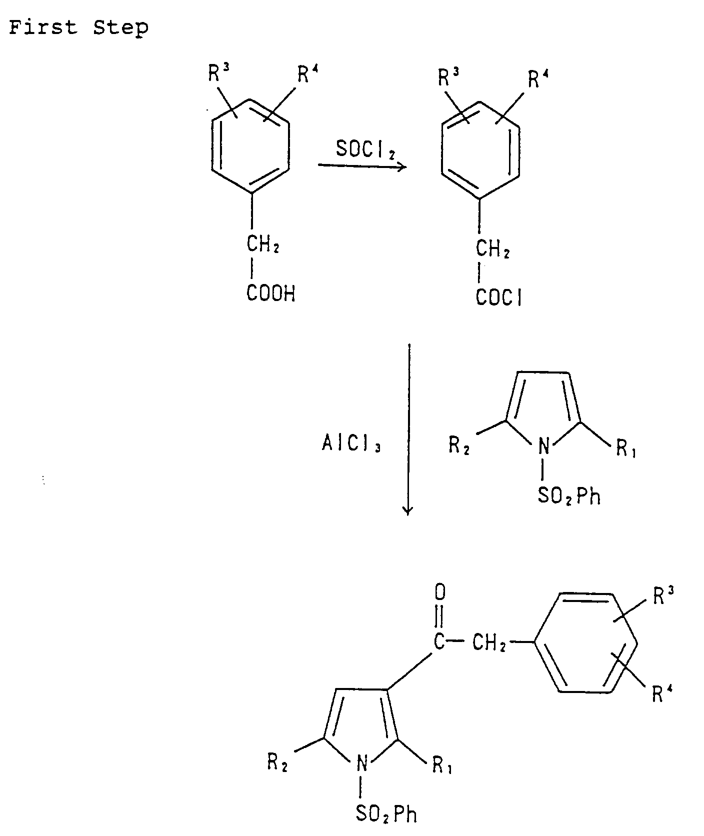
A substituted or non-substituted phenylacetic acid derivative is treated with, for example, thionyl chloride to afford the corresponding acid chloride, and the resulting acid chloride and a 1-benzenesulfonylpyrrole derivative (prepared according to the method described in Tetrahedron Letters 4901 (1981) or its analogous processes) are subjected to Friedel-Crafts reaction to give a 1-benzenesulfonyl-3-(phenylacetyl)pyrrole derivative. 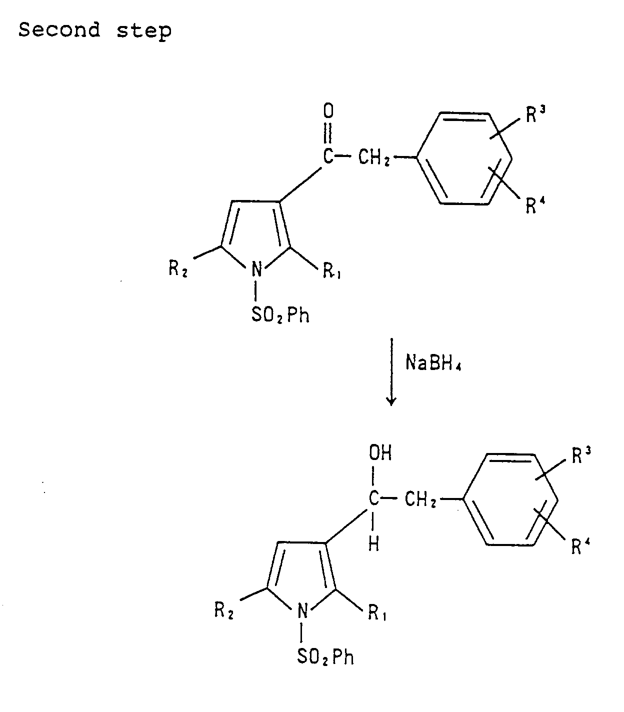
The resulting 1-benzenesulfonyl-3-(phenylacetyl)pyrrole derivative is reduced with, for example, sodium borohydride (NaBH₄) to give the corresponding 1-(benzenesulfonylpyrrol-3-yl)-2-(4-phenyl)ethanol derivative. 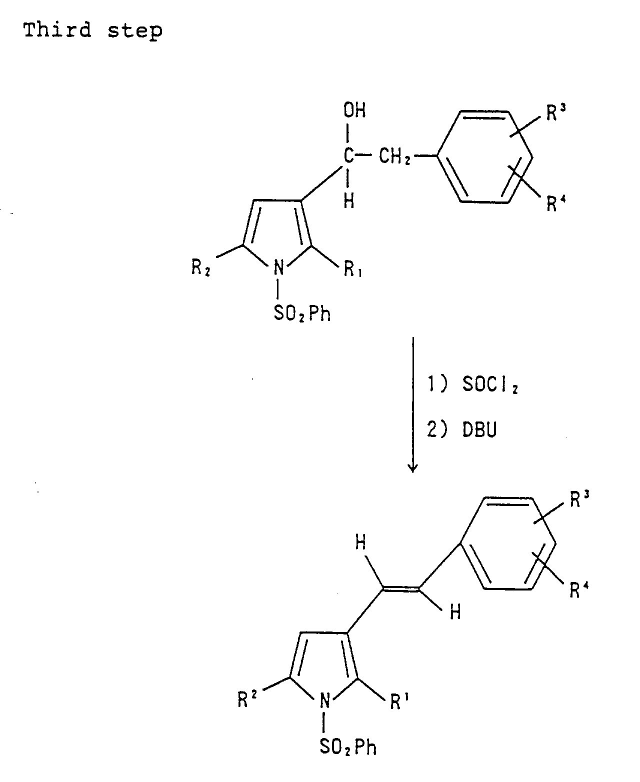
The resulting ethanol derivative is subjected to chlorination of its hydroxyl group using , for example, thionyl chloride, and then the result is further subjected to de-hydrochlorination reaction using 1,8-diazabicyclo[5,4,0]-7-undecene (DBU) to give 1-benzenesulfonyl-3-[2-(phenyl)-trans-ethenyl]pyrrole derivative. 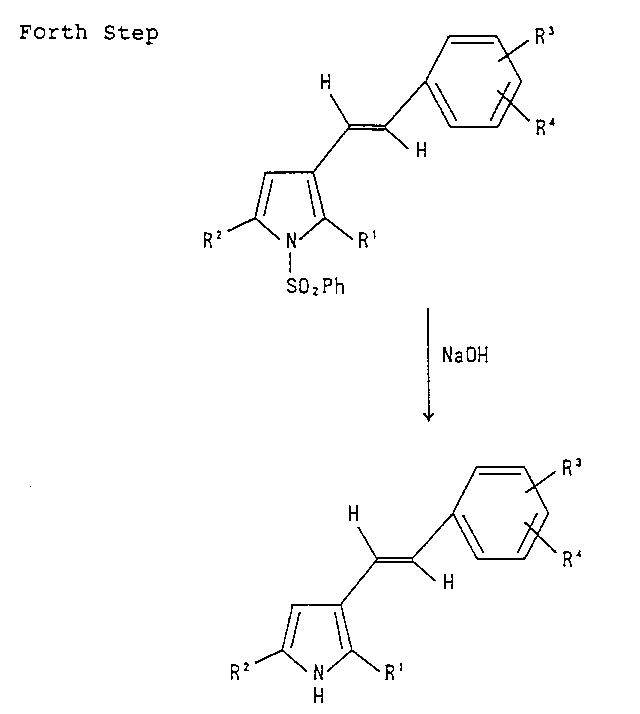
The aforementioned 1-benzenesulfonylpyrrole derivative is subjected to hydrolysis using, for example, sodium hydroxide to give compound of fomrula (3). Examples of the compound of the present invention represented by formula (3) will be set out below.
3-[2-(4-Chlorophenyl)-trans-ethenyl]pyrrole
m.p. 155-156°C (pale brown powder)
IR (cm ⁻¹, KBr): 3465, 3362, 1637, 1486, 1077, 967
NMR (ppm, CDCl₃): 6.4-6.6 (1H, m), 6.72 (1H, d), 7.05 (1H, d), 6.7-7.0 (2H, m), 7.28 (2H, d), 7.34 (2H, d), 7.8-8.5 (1H, b)
3-[2-(4-Nitrophenyl)-trans-ethenyl]pyrrole
m.p. 240-245°C (yellow powder)
IR (cm ⁻¹, KBr): 3365, 1629, 1588, 1492, 1329, 963, 788
NMR (ppm, CDCl₃): 6.3-6.5 (1H, m), 6.6-6.8 (1H, m), 6.9-7.1 (1H, m), 6.77 (1H, d), 7.30 (1H, d), 7.56 (1H, d), 8.14 (1H, d)
3-[2-(4-Bromophenyl)-trans-ethenyl]pyrrole
m.p. 164-167°C (colorless powder)
IR (cm ⁻¹, KBr): 3459, 3364, 1634, 1484, 1067, 966, 813
NMR (ppm, CDCl₃ ): 6.4-6.6 (1H, m), 6.68 (1H, d), 6.7-6.9 (1H, m), 6.8-7.0 (1H, m), 7.07 (1H, d), 7.27 (2H, d), 7.45 (2H, d), 8.0-8.5 (1H, b)
3-[2-(3-Chlorophenyl)-trans-ethenyl]pyrrole
m.p. 85-86°C (colorless powder)
IR (cm ⁻¹ KBr): 3457, 1634, 1419, 965, 786
NMR (ppm, CDCl₃ ): 6.4 -6.6 (1H, m), 6.6-7.6 (8H, m), 7.8-8.6 (1H, b)
3-[2-(2-Chlorophenyl)-trans-ethenyl]pyrrole
pale yellow oil
IR (cm ⁻¹ neat): 3437, 1631, 1468, 960, 747
NMR (ppm, CDCl₃ ) : 6.4 -6.6 (1H, m) , 6.6-7.8 (8H, m), 7.8-8.6 (1H, b)
3-[2-(4-Fluorophenyl)-trans-ethenyl]pyrrole
m.p. 128-130°C (pale brown powder)
IR (cm ⁻¹ , KBr): 3479, 1638, 1507, 1220, 1058, 966
NMR (ppm, CDCl₃ ): 6.4-6.6 (1H, m), 6.5-7.7 (8H, m), 7.7-8.5 (1H, b)
3-[2-(2,4-Dichlorophenyl)-trans-ethenyl]pyrrole
pale brown oil
NMR (ppm, CDCl₃ ): 6.4-6.6 (1H, m), 6.7-6.9 (1H, m), 6.9-7.7 (6H, m), 7.8-8.6 (1H, b)
3-[2-(3,4-Dichlorophenyl)-trans-ethenyl]pyrrole
m.p. 146-149°C (pale yellow powder)
IR (cm ⁻¹ KBr): 3445, 1633, 1425, 1133, 965, 781
NMR (ppm, CDCl₃ ): 6.4-6.6 (1H, m), 6.5-7.6 (7H, m), 7.8-8.5 (1H, b)
3-[2-(2,6-Dichlorophenyl)-trans-ethenyl]pyrrole
pale brown oil
IR (cm ⁻¹, neat): 3434, 1638, 1554, 1420, 1062, 963, 774
NMR (ppm, CDCl₃ ) : 6.5-6.7 (1H, m), 6.7-7.7 (7H, m), 7.7-8.6 (1H, b)
3-[2-(4-Trifluoromethylphenyl)-trans-ethenyl]pyrrole
m.p. 181-183°C (colorless powder)
IR (cm ⁻¹, KBr): 3492, 1638, 1329, 1104, 959, 828
NMR (ppm, CDCl₃ ): 6.4-6.6 (1H, m), 6.7-6.9 (1H, m), 6.9-7.1 (1H, m), 6.79 (1H, d), 7.17 (1H, d), 7.53 (4H, m), 7.7-8.5 (1H, b)
3-[2-(4-Methylphenyl)-trans-ethenyl]pyrrole
m.p. 135-140°C (colorless powder)
IR (cm ⁻¹ , KBr): 3416, 3887, 1632, 1510, 1417, 1057, 965, 812
NMR (ppm, CDCl₃ ): 2.33 (3H, s), 6.3-6.6 (1H, m), 6.6-7.5 (8H, m), 7.7-8.4 (1H, b)
3-[2-(4-tert-Butylphenyl)-trans-ethenyl]pyrrole
m.p. 117-121°C (colorless powder)
IR (cm ⁻¹, KBr): 3441, 2961, 1638, 1418, 1059, 964
NMR (ppm, CDCl₃ ): 3.26 (3H, s), 6.4-6.6 (1H, m), 6.6-7.5 (8H, m), 7.8-8.4 (1H, b)
3-[2-(4-Methoxyphenyl)-trans-ethenyl]pyrrole
m.p. 131-134°C (colorless powder)
IR (cm ⁻¹, CDCl₃ ): 3399, 1605, 1508, 1239, 1027, 964, 822
NMR (ppm, CDCl₃ ) : 3.81 (3H, s), 6.4-6.6 (1H, m), 6.6-7.5 (8H, m), 7.8-8.4 (1H, b)
3-[2-(4-n-Hexyloxyphenyl)-trans-ethenyl]pyrrole
m.p. 108-111°C (colorless powder)
IR (cm ⁻¹ , KBr) : 3437, 2931, 1602, 1509, 1252, 970, 824
NMR (ppm, CDCl₃ ): 0.7-2.0 (11H, m), 3.96 (2H, t), 6.3-6.6 (1H, m), 6.6-7.5 (6H, m), 7.9-8.4 (1H, b)
3-[2-[4-(2,2,2-Trifluoroethyl)oxyphenyl]-trans-ethenyl]pyrrole
m.p. 187-191°C (colorless powder)
IR (cm ⁻¹, KBr): 3325, 1639, 1508, 1282, 1173, 965
NMR (ppm, CDCl₃ ): 4.34 (1H, q), 6.4-6.6 (1H,m), 6.6-7.6 (8H, m), 7.8-8.6 (1H, b)
3-[2-[4-(2,2,3,3-Tetrafluoropropyl)oxyphenyl]-trans-ethenyl]pyrrole
m.p. 168-171°C (colorless powder)
IR (cm ⁻¹, KBr): 3366, 1509, 1253, 1124, 964
NMR (ppm, CDCl₃ ): 4.34 (2H, tt), 6.07 (1H, tt), 6.4-6.6 (1H, m), 6.6-7.5 (8H, m), 7.8-8.4 (1H, b)
3-[2-(4-biphenyl)-trans-ethenyl]pyrrole
m.p. 170-180°C (colorless powder)
IR (cm ⁻¹, KBr): 3438, 3383, 1634, 1484, 1422, 965
NMR (ppm, CDCl₃ ): 6.4-6.6 (1H, m), 6.7-6.9 (1H, m), 6.9-7.8 (12H, m), 7.9-8.5 (1H, b)
3-[2-[4-(N,N-Dimethylamino)phenyl]-trans-ethenyl)pyrrole
m.p. 169-171°C (brown powder)
IR (cm ⁻¹, KBr): 3413, 1604, 1518, 1357, 1061, 961, 813
NMR (ppm, CDCl₃ ): 2.95 (6H, s), 6.3-6.6 (1H, m), 6.6-7.5 (8H, m), 7.8-8.3 (1H, b)
MASS: 212 (M⁺ )
3-[2-(4-Methylthiophenyl)-trans-ethenyl]pyrrole
m.p. 143-144°C (colorless powder)
IR (cm ⁻¹, KBr): 3413, 1636, 1489, 1419, 961
NMR (ppm, CDCl₃ ): 2.49 (3H, m), 6.4-6.6 (1H, m), 6.7-7.1 (2H, m), 6.73 (1H, d), 7.05 (1H, d), 7.20 (2H, d), 7.38 (2H, d), 7.8-8.4 (1H, b)
2,5-Dimethyl-3-[2-(4-methylphenyl)-trans-ethenyl]pyrrole
m.p. 123-124°C (colorless powder)
IR (cm ⁻¹, KBr): 3382, 3332, 1632, 1436, 965, 792
NMR (ppm, CDCl₃ ): 2.22 (3H, m), 2.29 (3H, s), 2.48 (3H, s), 6.08 (1H, b), 6.58 (1H, d), 6.97 (1H, d), 7.20 (2H, d), 7.2-7.8 (1H, b)
MASS: 215 (M⁺ )

Antifungal activity of the compounds of the present invention represented by formula (I) was studied.

### (A) Evaluation of antifungal activity in vitro

Sample solutions were prepared by dissolving a compound to be tested in DMSO at 10 mg/ml and diluted with DMSO by two fold dilution method. Fluconazole was used as a reference antifungal drug. To a culture medium, autoclaved and then warmed at 45°C , 1/100 volume of sample solution, or alternatively, water or DMSO as reference was added and mixted, and then the culture medium was added to a plastic dish to form agar plates. A culture of Trichophyton rubrum IKD (1 × 10⁶ CFU/ml) was smeared at two portions of the agar plates with a width of 1-2 cm using Micro-Roop #10 (HWE, 1µ l). The agar plates inoculated were cultured at 27 °C for seven days, and then the plates were subjected to observation for determining MICs (minimum concentrations of drugs which completely prevents the growth of fungi as compared with the growth of fungi on reference plates containing water or DMSO). Results are summarized in Table 1 set out below. It can be understood that the compounds of the present invention have potent antifungal activities (0.39 to 6.3 µ g/ml) as compared to fluconazole having the MIC of 100µ g/ml.

**Table 1**

| COMPOUND | MIC (µ g/ml) |
|---|---|
| EXAMPLE 1 | 3.1 |
| EXAMPLE 10 | 3.1 |
| EXAMPLE 15 | 0.39 |
| EXAMPLE 18 | 6.3 |
| EXAMPLE 27 | 0.39 |
| EXAMPLE 29 | 0.39 |
| EXAMPLE 33 | 0.78 |
| EXAMPLE 34 | 0.39 |
| FLUCONAZOLE | 100 |

### (B) Evaluation of antifungal activity in vivo

4 × 10⁷ cells of Candida albicans were administered intravenously to a group of five Std/ddy male mice (body weigt of from 15 to 17 g) via tale vein. Soon after the inoculation of the bacteria, each test sample, which was prepared by dissolving the compound of the present invention in a mixture of water and polyethyleneglycole-200 and was adjusted to the amout of 10 mg/kg, was administered orally to the aminal. The test sample was administered once a day for successive five days. Death or aliveness of the aminals was then observed for 30 days after the inoculation of bacteria, and chemotherapeutic index of the compound was caluculated based on the avarage survival days. Fluconazole was used as positive reference compound for comparison with the compound of the present invention. The results are summarized in Table 2 set out below.

**Table 2**

| COMPOUND | CHEMOTHERAPEUTIC INDEX |
|---|---|
| EXAMPLE 1 | 132.0 |
| EXAMPLE 2 | 113.8 |
| EXAMPLE 3 | 98.9 |
| FLUCONAZOLE | 100.0 |

The aforementioned compounds of the present invention and their pharmacologically acceptable salts are useful as antifungal agents when administered orally or parenterally to an mammal, preferably to human patient. According to an preferred embodiment of the resent invention, there is provided a pharmaceutical composition comprising an effective amount of the compound of the present invention represented by formula (1) or a pharmacologically acceptable salt thereof together with a pharmaceutically acceptable carrier or coating.

The pharmaceutical composition comprising the compound of the present invention represented by formula (1) is useful as medicament for the treatment of antifungal infection. The pharmaceutical composition comprising said compound as an effective ingredient is usually prepared as orally available composition such as, for example, capsules, tablets, subtilized granules, granules, powder, or syrup, or parenterally used composition such as, for example, injection, suppository, eye drop, eye ointment, ear drop, or topical composition. These pharmaceutical compositions can be formulated according to ordinary processes and may be added with pharmacologically or pharmaceutically acceptable additives, if desired. Where the orally available pharmaceutical composition is used for the treatment of antifungal infection, about 5 - 500 mg of an active ingredient per day may usually be administered to an adult patient.

### EXAMPLES

The present invention will be hereinafter exaplained more specifically by way or examples. However, the present invetnion is not be limited to these examples.

### Example 1: 1-[3-[2-(4-Chlorophenyl)-trans-ethenyl]pyrrol-1-yl]-2-(2,4-difluorophenyl)-3-(1H-1,2,4-triazol-1-yl)propan-2-ol

0.85 g of 60% sodium hydride was suspended in 50 ml of N,N-dimethylformamide (DMF), and 4.32 g of 3-[2-(4-chlorophenyl)-trans-ethenyl]pyrrole prepared according to the aforementioned method was added to the suspension. The suspension was stirred for 30 minutes at room temperature, and then 5.04 g of 1-[2-(2,4-difluorophenyl)-2,3-epoxypropyl]-1H-1,2,4-triazole prepared by the method described in Japanese Patent Unexamined Publication No. (Sho)58-32868 (m.p. 55-60 °C ) was added and stirred at 90-100 °C for one hour. After the reaction was completed, the reaction mixture was poured into ice-water and extracted three times with ethyl acetate. The organic layer was washed with water and dried, and then the solvent was removed under a reduced pressure. The residural crystals were recrystallized from ethanol to give 6.30 g of the title compound (yield 67%).
m.p. 170-171°C (ethanol/coloerless prisms)
IR (cm ⁻¹ , KBr): 3124, 1512, 1498, 1274, 1131, 968, 850
NMR (ppm, CDCl₃ ): 4.19 (1H, d), 4.23 (2H, s), 4.88 (1H, d), 4.8-5.1 (1H, b), 6.2-6.4 (1H, m), 6.5-7.8 (12H, m), 7.81(1H, s), 7.88 (1H, s)
MASS: 440 (M⁺ )

### Example 2: 1-[3-[2-(4-Nitrophenyl)-trans-ethenyl]pyrrol-1-yl]-2-(2,4-difluorophenyl)-3-(1H-1,2,4-triazol-1-yl)propan-2-ol

By using 5.04 g of 3-[2-(4-nitrophenyl)-trans-ethenyl]pyrrole for 3-[2-(4-chlorophenyl)-trans-ethenyl]pyrrol in Example 1, 1.35 g of the title compound was obtained in the same manner as described above (yield 59%).
m.p. 170-171°C (ethanol/pale yellow prisms)
IR (cm ⁻¹ , KBr): 3133, 1630, 1590, 1508, 1339, 1111, 965
NMR (ppm, CDCl₃ ): 4.21 (1H, d), 4.25 (2H, s), 4.93 (1H, d), 4.7-5.2 (1H, b), 6.3-7.6 (8H, m), 7.49 (2H, d), 7.82 (1H, s), 7.89 (1H, s), 8.16 (2H, d)
MASS: 451 (M⁺ )

### Example 3: 1-[3-[2-(4-Bromophenyl)-trans-ethenyl]pyrrol-1-yl]-2-(2,4-difluorophenyl)-3-(1H-1,2,4-triazol-1-yl)propan-2-ol

Starting from 1.26 g of 3-[2-(4-bromophenyl)-trans-ethenyl]pyrrole, 1.35 g of the title compound was obtained in the same manner as described in Example 1 (yield 55%).
m.p. 180-183°C (ethanol/colorless prisms)
IR (cm ⁻¹ , KBr): 3124, 1638, 1618, 1498, 1274, 1138, 967, 773
NMR (ppm, CDCl₃ ): 4.18 (1H, d), 4.22 (2H, s), 4.88 (1H, d), 4.8-5.0 (1H, b), 6.2-6.4 (1H, m), 6.4-7.7 (11H, m), 7.81 (1H, s), 7.88 (1H, s)
MASS: 485 (M ⁺ )

### Example 4: 1-[3-[2-(3-Chlorophenyl)-trans-ethenyl]pyrrol-1-yl]-2-(2,4-difluorophenyl)-3-(1H-1,2,4-triazol-1-yl)propan-2-ol

By using 1.03 g of 3-[2-(3-chlorophenyl)-trans-ethenyl]pyrrole, 1.25 g of the title compound was obtained (yield 56%).
m.p. 133-135°C (ethanol/white powder)
IR (cm ⁻¹ , KBr): 3132, 1641, 1420, 1274, 1083, 966
NMR (ppm, CDCl₃ ): 4.19 (1H, d), 4.22 (2H, s), 4.87 (1H, d), 4.6-5.1 (1H, b), 6.2-6.4 (1H, m), 6.5-7.6 (1H, m), 7.80 (1H, s), 7.87 (1H, s)
MASS: 440 (M⁺ )

### Example 5: 1-[3-[2-(2-Chlorophenyl)-trans-ethenyl]pyrrol-1-yl]-2-(2,4-difluorophenyl)-3-(1H-1,2,4-triazol-1-yl)propan-2-ol

By using 1.03 g of 3-[2-(2-chlorophenyl)-trans-ethenyl]pyrrole, 0.56 g of the title compound was obtained (yield 25%).
m.p. 120-122°C (ethanol/colorless prisms)
IR (cm ⁻¹ , KBr): 3129, 1637, 1618, 1275, 968
NMR (ppm, CDCl₃ ): 4.20 (1H, d), 4.27 (2H, s), 4.89 (1H, d), 4.7-5.2 (1H, b), 6.3-6.5 (1H, m), 6.5-6.7 (1H, m), 6.7-7.7 (10H, m), 7.81 (1H, s), 7. 89(1H, s)
MASS: 440 (M⁺ )

### Example 6: 1-[3-[2-[4-Fluorophenyl)-trans-ethenyl]pyrrol-1-yl]-2-(2,4-difluorophenyl)-3-(1H-1,2,4-triazol-1-yl)propan-2-ol

By using 1.30 g of 3-[2-(4-fluorophenyl)-trans-ethenyl]pyrrole, 0.97 g of the title compound was obtained (yield 33%).
m.p. 170-172°C (ethanol/colorless prisms)
IR (cm ⁻¹ , KBr): 3126, 1618, 1508, 1498, 1274, 1236, 1139, 968
NMR (ppm, CDCl₃ ): 4.19 (1H, d), 4.23 (2H, s), 4.88 (1H, d), 4.7-5.1 (1H, b), 6.2-6.4 (1H, m), 6.5-7.6 (11H, m), 7.80 (1H, s) ,7.88 (1H, s)
MASS: 424 (M⁺ )

### Example 7: 1-[3-[2-(2,4-Dichlorophenyl)-trans-ethenyl]pyrrol-1-yl]-2-(2,4-difluorophenyl)-3-(1H-1,2,4-triazol-1-yl)propan-2-ol

By using 1.20 g of 3-[2-(2,4-dichlorophenyl)-trans-ethenyl]pyrrole, 1.40 g of the title compound was obtained (yield 58%).
m.p. 85-93°C (2-propanol/colorless prisms)
IR (cm ⁻¹ , KBr): 3108, 1630, 1503, 1275, 965, 850
NMR (ppm, CDCl₃ ): 4.21 (1H, d), 4.24 (2H, s), 4.89 (1H, d), 6.3-6.5 (1H, b), 6.5-7.6 (10H, m), 7.81 (1H, s), 7.91 (1H, s)
MASS: 475 (M⁺ )

### Example 8: 1-[3-[2-(3,4-Dichlorophenyl)-trans-ethenyl]pyrrol-1-yl]-2-(2,4-difluorophenyl)-3-(1H-1,2,4-triazol-1-yl)propan-2-ol

By using 1.20 g of 3-[2-(3,4-dichlorophenyl)-trans-ethenyl]pyrrole, 0.60 g of the title compound was obtained (yield 25%).
m.p. 140-142°C (2-propanol/colorless prisms)
IR (cm ⁻¹ , KBr): 3125, 1617, 1499, 1273, 1139, 967, 853, 619
NMR (ppm, CDCl₃ ): 4.20 (1H, d), 4.23 (2H, s), 4.88 (1H, d), 4.7-5.2 (1H, b), 6.2-6.4 (1H, m), 6.4-7.6 (10H, m), 7.81 (1H, s), 7.89 (1H, s)
MASS: 475 (M⁺ )

### Example 9: 1-[3-[2-(2,6-Dichlorophenyl)-trans-ethenyl]pyrrol-1-yl]-2-(2,4-difluorophenyl)-3-(1H-1,2,4-triazol-1-yl)propan-2-ol

By using 1.20 g of 3-[2-(2,6-dichlorophenyl)-trans-ethenyl]pyrrole, 1.65 g of the title compound was obtained (yield 69%).
m.p. 144-147°C (ethanol/colorless prisms)
IR (cm ⁻¹ , KBr): 3127, 1618, 1507, 1422, 1140, 967, 776
NMR (ppm, CDCl₃ ): 4.21 (1H, d), 4.24 (2H, s), 4.89 (1H, d), 4.92 (1H, b), 6.3-6.5 (1H, m), 6.6-7.6 (10H, m), 7.81 (1H, s), 7.88 (1H, s)
MASS: 475 (M⁺ )

### Example 10: 1-[3-[2-(4-Trifluoromethylphenyl)-trans-ethenyl]pyrrol-1-yl]-2-(2,4-difluorophenyl)-3-(1H-1,2,4-triazol-1-yl)propan-2-ol

By using 0.33 g of 3-[2-(4-trifluoromethylphenyl)-trans-ethenyl]pyrrole, 0.49 g of the title compound was obtained (yield 74%).
m.p. 162-163°C (2-propanol/colorless prisms)
IR (cm ⁻¹ , KBr): 3128, 1613, 1322, 1166, 1133, 1116, 968, 855, 619
NMR (ppm, CDCl₃ ): 4.20 (1H, d), 4.24 (2H, s), 4.89 (1H, s), 4.7-5.2 (1H, b), 6.3-7.7 (12H, m), 7.82 (1H, s), 7.89 (1H, s)
MASS: 474 (M⁺ )

### Example 11: 1-[3-[2-(4-Methylphenyl)-trans-ethenyl]pyrrol-1-yl]-2-(2,4-difluorophenyl)-3-(1H-1,2,4-triazol-1-yl)propan-2-ol

By using 0.93 g of 3-[2-(4-methylphenyl)-trans-ethenyl]pyrrole, 0.98 g of the title compound was obtained (yield 47%).
m.p. 159-162°C (ethanol/colorless prisms)
IR (cm ⁻¹ , KBr): 3122, 1618, 1510, 1498, 1419, 1274, 1139, 967, 618
NMR (ppm, CDCl₃ ): 2.33 (3H, s), 4.19 (1H, d), 4.22 (2H, s), 4.89 (1H, d), 6.2-6.4 (1H, m), 6.5-7.6 (11H, m), 7.81 (1H, s), 7.89 (1H, s)
MASS: 420 (M⁺ )

### Example 12: 1-[3-[2-(4-t-Butylphenyl)-trans-ethenyl]pyrrol-1-yl]-2-(2,4-difluorophenyl)-3-(1H-1,2,4-triazol-1-yl)propan-2-ol

By using 1.14 g of 3-[2-(4-t-butylphenyl)-trans-ethenyl]pyrrole, 1.26 g of the title compound was obtained (yield 54%).
m.p. 169-172°C (ethanol/colorless prisms)
IR (cm ⁻¹ , KBr): 3124, 2963, 1618, 1498, 1418, 1273, 1140, 967, 617
NMR (ppm, CDCl₃ ): 1.32 (9H, s), 4.22 (1H, d), 4.89 (1H, d), 6.2-6.5 (1H, m), 6.5-7.7 (11H, m), 7.82 (1H, s), 7.94 (1H, s)
MASS: 462 (M⁺ )

### Example 13: 1-[3-[2-(4-Methoxyphenyl)-trans-ethenyl]pyrrol-1-yl]-2-(2,4-difluorophenyl)-3-(1H-1,2,4-triazol-1-yl)propan-2-ol

By using 0.54 g of 3-[2-(4-methoxyphenyl)-trans-ethenyl]pyrrole, 0.37 g of the title compound was obtained (yield 38%).
m.p. 141-143°C (2-propanol/colorless prisms)
IR (cm ⁻¹ , KBr): 3422, 3124, 1509, 1248, 1138, 967, 618
NMR (ppm, CDCl₃ ): 3.81 (3H, s), 4.19 (1H, d), 4.22 (2H, s), 4.6-5.1 (1H, b), 4.88 (1H, d), 6.2-6.4 (1H, m), 6.5-7.6 (11H, m), 7.80 (1H, s), 7.88 (1H, s)
MASS: 436 (M⁺ )

### Example 14: 1-[3-[2-(4-n-Hexyloxyphenyl)-trans-ethenyl]pyrrol-1-yl]-2-(2,4-difluorophenyl)-3-(1H-1,2,4-triazol-1-yl)propan-2-ol

By using 0.49 g of 3-[2-(4-n-hexyloxyphenyl)-trans-ethenyl]pyrrole, 0.43 g of the title compound was obtained (yield 46%).
m.p. 148-150°C (ethanol/colorless prisms)
IR (cm ⁻¹ , KBr): 3123, 2954, 1509, 1250, 1139, 968, 852, 617
NMR (ppm, CDCl₃ ): 0.7-1.0 (3H, m), 1.0-2.0 (8H, m), 3.95 (2H, t), 4.19 (1H, d), 4.88 (1H, d), 4.22 (2H, s), 6.2-6.4 (1H, m), 6.5-7.6 (11H, m), 7.80 (1H, s), 7.88 (1H, s)
MASS: 506 (M⁺ )

### Example 15: 1-[3-[2-[4-(2,2,2-Trifluoroethyl)oxyphenyl]-trans-ethenyl]pyrrol-1-yl]-2-(2,4-difluorophenyl)-3-(1H-1,2,4-triazol-1-yl)propan-2-ol

By using 0.65 g of 3-[2-[4-(2,2,3-trifluoroethyl)oxyphenyl]-trans-ethenyl]pyrrole, 0.66 g of the title compound was obtained (yield 54%).
m.p. 168-170°C (ethanol/colorless prisms)
IR (cm ⁻¹ , KBr): 3429, 3120, 1619, 1508, 1236, 1169, 967
NMR (ppm, CDCl₃ ): 4.19 (1H, d), 4.22 (2H, s), 4.34 (2H, g), 4.6-5.1 (1H, b), 4.88 (1H, d) , 6.2-6.4 (1H, m), 6.5-7.6 (11H, m), 7.80 (1H, s), 7.88 (1H, s)
MASS: 504 (M⁺ )

### Example 16: 1-[3-[2-[4-(2,2,3,3-Tetrafluoropropyl)oxyphenyl]-trans-ethenyl)pyrrol-1-yl]-2-(2,4-difluorophenyl)-3-(1H-1,2,4-triazol-1-yl)propan-2-ol

By using 0.95 g of 3-[2-[4-(2,2,3,3-tetrafluoropropyl) oxyphenyl]-trans-ethenyl]pyrrole, 0.99 g of the title compound was obtained (yield 58%).
m.p. 159-161°C (ethanol/white powder)
IR (cm ⁻¹ , KBr): 3429, 3122, 1617, 1509, 1274, 1117, 966, 859, 618
NMR (ppm, CDCl₃ ): 4.19 (1H, d), 4.23 (2H, s), 4.34 (2H, t, t), 4.88 (1H, d), 6.07 (1H, t, t), 6.2-6.5 (1H, m), 6.5-7.7 (11H, m), 7.81 (1H, s), 7.89 (1H, s)
MASS: 536 (M⁺ )

### Example 17: 1-[3-[2-(4-Biphenyl)-trans-ethenyl]pyrrol-1-yl]-2-(2,4-difluorophenyl)-3-(1H-1,2,4-triazol-1-yl)propan-2-ol

By using 1.24 g of 3-[2-(4-biphenyl)-trans-ethenyl]pyrrole, 1.59 g of the title compound was obtained (yield 65%).
m.p. 205-206°C (ethanol/colorless prisms)
IR (cm ⁻¹ , KBr): 3429, 3119, 1618, 1486, 1419, 1273, 1140, 966, 767
NMR (ppm, CDCl₃ ): 4.23 (1H, d), 4.24 (2H, s), 4.90 (1H, d), 6.3-6.5 (1H, m), 6.5-8.0 (16H, m), 7.82 (1H, s), 7.95 (1H, s)
MASS: 482 (M⁺ )

### Example 18: 1-[3-[2-[4-(N,N-Dimethyl)aminophenyl]-trans-ethenyl]pyrrol-1-yl]-2-(2,4-difluorophenyl)-3-(1H-1,2,4-triazol-1-yl)propan-2-ol

By using 0.30 g of 3-[2-[4-(N,N-dimethyl)aminophenyl]-trans-ethenyl]pyrrole, 0.36 g of the title compound was obtained (yield 57%).
m.p. 158-160°C (ethanol/colorless prisms)
IR (cm ⁻¹ , KBr): 3119, 1609, 1515, 1350, 967, 617
NMR (ppm, CDCl₃ ): 2.95 (6H, s), 4.19 (1H, d), 4.21 (2H, s), 4.82 (1H, b), 4.89 (1H, d), 6.2-6.4 (1H, m), 6.4-7.7 (11H, m), 7.80 (1H, s), 7.88 (1H, s)
MASS: 449 (M⁺ )

### Example 19: 1-[3-[2-(4-Aminophenyl)-trans-ethenyl]pyrrol-1-yl]-2-(2,4-difluorophenyl)-3-(1H-1,2,4-triazol-1-yl)propan-2-ol

0.97 g of 1-[3-[2-(4-nitrophenyl)-trans-ethenyl]pyrrol-1-yl]-2-(2,4-difluorophenyl)-3-(1H-1,2,4-triazol-1-yl)propan-2-ol obtained in Example 2 was subjected to an ordinary reduction reaction using iron powder and hydrochloric acid to afford 0.78 g of the title compound (yield 86%).
m.p. 99-101 °C (toluene/colorless prisms)
IR (cm ⁻¹ , KBr): 3439, 3350, 1619, 1513, 1275, 1141, 968
NMR (ppm, CDCl₃ ): 4.19 (1H, d), 4.21 (2H, s), 4.87 (1H, d), 6.2-6.4 (1H, m), 6.5-7.7 (11H, m), 7.80 (1H, s), 7.88 (1H, s)
MASS: 421 (M⁺ )

### Example 20: 1-[3-[2-(4-Acetoaminophenyl)-trans-ethenyl]pyrrol-1-yl]-2-(2,4-difluorophenyl)-3-(1H-1,2,4-triazol-1-yl)propan-2-ol

0.20 g of 1-[3-[2-(4-aminophenyl)-trans-ethenyl]pyrrol-1-yl]-2-(2,4-difluorophenyl)-3-(1H-1,2,4-triazol-1-yl)propan-2-ol obtained in Example 19 in dichloromethane was treated with acetyl chloride in the presense of saturated aqueous sodium bicarbonate solution to afford 0.135 g of the title compound (yield 61%).
m.p. 90-107 °C (ethyl acetate-methanol/pale yellow powder)
IR (cm ⁻¹ , KBr): 3294, 1667, 1597, 1509, 1272, 965
NMR (ppm, CDCl₃ ): 2.16 (3H, s), 4.19 (1H, d), 4.22 (2H, s), 4.88 (1H, d), 4.7-5.0 (1H, b), 6.2-6.4 (1H, m), 6.3-7.7 (11H, m), 7.80 (1H, s), 7.88(1H, s)
MASS: 463 (M⁺ )

### Example 21: 1-[3-[2-(4-Methylthiophenyl)-trans-ethenyl]pyrrol-1-yl]-2-(2,4-difluorophenyl)-3-(1H-1,2,4-triazol-1-yl)propan-2-ol

By using 0.07 g of 3-[2-(4-methylthiophenyl)-trans-ethenyl]pyrrole, 0.07 g of the title compound was obtained (yield 48%).
m.p. 153-155°C (2-propanol/colorless prisms)
IR (cm ⁻¹ , KBr): 3124, 1618, 1498, 1274, 1140, 967
NMR (ppm, CDCl₃ ): 2.48 (3H, s), 4.23 (2H, s), 4.22 (1H, d), 4.89(1H, d), 6.2-6.4 (1H, m), 6.5-7.6 (11H, m), 7.82 (1H, s), 7.96 (1H, s)
MASS: 452 (M⁺ )

### Example 22: 1-[3-[2-(4-Chlorophenyl)-trans-ethenyl]pyrrol-1-yl]-2-(4-chlorophenyl)-3-(1H-1,2,4-triazol-1-yl)propan-2-ol

By using 1.05 g of 3-[2-(4-chlorophenyl)-trans-ethenyl]pyrrole obtained above and 1.26 g of 1-[2-(4-chlorophenyl)-2,3-epoxypropyl]-1H-1,2,4-triazole prepared by the method described in Japanese Patent Unexamined Publication No. (Sho)57-14575 as starting materials, 1.35 g of the title compound was obtained (yield 60%).
m.p. 108-110°C (ethanol/colorless prisms)
IR (cm ⁻¹ , KBr): 3180, 1636, 1509, 1489, 1093, 1079, 814
NMR (ppm, CDCl₃ ): 4.06 (1H, d), 4.26 (1H, d), 4.29 (1H, d), 4.58(1H, d), 6.2-6.4 (1H, m), 6.4-6.6 (1H, m), 6.6-6.7 (1H, m), 6.65 (1H, d), 6.94 (1H, d), 7.1-7.5 (8H, m), 7.84 (1H, s), 7.85(1H, s)
MASS: 439 (M⁺ )

### Example 23: 1-[3-[2-(4-Chlorophenyl)-trans-ethenyl]pyrrol-1-yl]-2-(2,4-dichlorophenyl)-3-(1H-1,2,4-triazol-1-yl)propan-2-ol

By using 0.91 g of 3-[2-(4-chlorophenyl)-trans-ethenyl]pyrrole obtained above and 1.20 g of 1-[2-(2,4-dichlorophenyl)-2,3-epoxypropyl]-1H-1,2,4-triazole prepared by the method described in Japanese Patent Unexamined Publication No. (Sho)57-14575, 0.50 g of the title compound was obtained (yield 24%).
m.p. 191-192°C (ethanol/colorless prisms)
IR (cm ⁻¹ , KBr): 3124, 1637, 1489, 1273, 1100, 969, 814
NMR (ppm, CDCl₃ ): 4.14 (1H, d), 4.33 (1H, d), 4.52(1H, d), 5.35(1H, b), 5.43 (1H, d), 6.2-6.4 (1H, m), 6.5-7.7 (11H, m), 7.84 (1H, s), 7.90 (1H, s)
MASS: 473 (M⁺ )

### Example 24: 1-[3-[2-(4-Bromophenyl)-trans-ethenyl]pyrrol-1-yl]-2-(2,4-dichlorophenyl)-3-(1H-1,2,4-triazol-1-yl)propan-2-ol

By using 1.26 g of 3-[2-(4-bromophenyl)-trans-ethenyl]pyrrole obtained above and 1.37 g of 1-[2-(2,4-dichlorophenyl)-2,3-epoxypropyl]-1H-1,2,4-triazole used in Example 23, 0.68 g of the title compound was obtained (yield 26%).
m.p. 190-192°C (ethanol/white powder)
IR (cm ⁻¹ , KBr): 3124, 1637, 1509, 1486, 1274, 1128, 1073, 810
NMR (ppm, CDCl₃ ): 4.14 (1H, d), 4.33 (1H, d), 4.52 (1H, d), 5.35 (1H, b), 5.42 (1H, d), 6.2-6.5 (1H, m), 6.5-7.7 (11H, m), 7.80 (1H, s), 7.90 (1H, s)
MASS: 518 (M⁺ )

### Example 25: 1-[3-[2-(4-Chlorophenyl)-trans-ethenyl]pyrrol-1-yl]-2-(4-trifluoromethylphenyl)-3-(1H-1,2,4-triazol-1-yl)propan-2-ol

By using 0.80 g of 3-[2-(4-chlorophenyl)-trans-ethenyl]pyrrole obtained above and 1.26 g of 1-[2-(4-trifluoromethylphenyl)-2,3-epoxypropyl]-1H-1,2,4-triazole prepared by the method described in Japanese Patent Unexamined Publication No. (Hei)3-72468, 0.49 g of the title compound was obtained (yield 26%).
m.p. 111-114°C (ethanol/colorless prisms)
IR (cm ⁻¹ , KBr): 3224, 1636, 1329, 1161, 1127, 1071
NMR (ppm, CDCl₃ ): 4.09 (1H, d), 4.29 (1H, d), 4.34 (1H, d), 4.65 (1H, d), 6.3-6.4 (1H, m), 6.4-6.6 (1H, m), 6.6-6.8 (1H, m), 6.65(1H, d), 6.94 (1H, d), 7.1-7.7 (8H, m), 7.93 (1H, s), 7.94 (1H, s)
MASS: 472 (M⁺ )

### Example 26: 1-[2,5-Dimethyl-3-[2-(4-methylthio)phenyl]-trans-ethenyl]pyrrol-1-yl]-2-(2,4-difluorophenyl)-3-(1H-1,2,4-triazol-1-yl)propan-2-ol

By using 0.82 g of 2,5-Dimethyl-3-[2-(4-methylthio)phenyl]-trans-ethenyl]pyrrole, 0.332 g of the title compound was obtained (yield 20%).
m.p. 69-72°C (pale yellow powder)
IR (cm ⁻¹ , KBr): 2922, 1630, 1618, 1500, 1427, 1274, 965
NMR (ppm, CDCl₃ ): 2.14 (3H, s), 2.20 (3H, s), 2.48 (3H, s), 4.12 (1H, d), 4.30 (1H, d), 4.32 (1H, d), 4.95 (1H, d), 6.16 (1H, s), 6.4-7.7 (7H, m), 6.60 (2H, d), 6.97 (2H, d), 7.80 (1H, s), 7.94 (1H s)
MASS: 480 (M⁺ )

### Example 27: 1-[2-Chloro-3-[2-(4-chlorophenyl)-trans-ethenyl]pyrrol-1-yl]-2-(2,4-difluorophenyl)-3-(1H-1,2,4-triazol-1-yl)propan-2-ol

By treating 0.30 g of 1-[3-[2-(4-chlorophenyl)-trans-ethenyl]pyrrol-1-yl]-2-(2,4-difluorophenyl)-3-(1H-1,2,4-triazol-1-yl)propan-2-ol obtained in Example 1 with 0.10 g of N-chlorosuccinimide (NCS) in N,N-dimethylformamide (DMF), 0.15 g of the title compound was obtained (yield 46%).
m.p. 180-185°C (ethanol/colorless prisms)
IR (cm ⁻¹ , KBr): 3430, 1619, 1488, 966
NMR (ppm, CDCl₃): 4.10 (1H, d), 4.16 (1H, d), 4.52 (1H, d), 5.02 (1H, d), 6.41 (1H, d), 6.6-7.7 (8H, m), 7.80 (1H, s), 7.93 (1H, s)
MASS: 475 (M⁺ )

### Example 28: 1-[2-Chloro-3-[2-(4-methylphenyl)-trans-ethenyl]pyrrol-1-yl]-2-(2,4-difluorophenyl)-3-(1H-1,2,4-triazol-1-yl)propan-2-ol

By using 0.84 g of 1-[3-[2-(4-methylphenyl)-trans-ethenyl]pyrrol-1-yl]-2-(2,4-difluorophenyl)-3-(1H-1,2,4-triazol-1-yl)propan-2-ol obtained in Example 11 as starting material, 0.41 g of the title compound was obtained in the same manner as Example 27 (yield 45%).
m.p. 149-151°C (2-propanol/colorless needles)
IR (cm ⁻¹ , KBr): 1618, 1497, 1272, 966, 853
NMR (ppm, CDCl₃ ): 2.34 (3H, s), 4.04 (1H, d), 4.15 (1H, d), 4.53 (1H, d), 5.03 (1H, d), 6.42 (1H, d), 6.5-7.7 (10H, m), 7.80 (1H, s), 7.92(1H, s)
MASS: 454 (M⁺ )

### Example 29: 1-[2-Chloro-3-[2-[4-(2,2,3,3-tetrafluoropropyl) oxyphenyl]-trans-ethenyl]pyrrol-1-yl]-2-(2,4-difluorophenyl)-3-(1H-1,2,4-triazol-1-yl)propan-2-ol

By using 0.30 g of 1-[3-[2-[4-(2,2,3,3-tetrafluoropropyl) oxyphenyl]-trans-ethenyl]pyrrol-1-yl]-2-(2,4-difluorophenyl)-3-(1H-1,2,4-triazol-1-yl)propan-2-ol obtained in Example 16 as starting material, 0.15 g of the title compound was obtained in the same manner as Example 27 (yield 47%).
m.p. 130-134°C (ethanol/colorless prisms)
IR (cm ⁻¹ , KBr): 1618, 1500, 1110, 966
NMR (ppm, CDCl₃ ): 3.9-5.3 (6H, m), 6.07 (1H, t, t), 6.40 (1H, d), 6.3-7.6 (10H, m), 7.83 (1H, s), 8.03 (1H, s)
MASS: 570 (M⁺ )

### Example 30: 1-[2,5-Dichloro-3-[2-(4-chlorophenyl)-trans-ethenyl]pyrrol-1-yl]-2-(4-trifluoromethylphenyl)-3-(1H-1,2,4-triazol-1-yl)propan-2-ol

By treating 0.80 g of 1-[3-[2-(4-chlorophenyl)-trans-ethenyl]pyrrol-1-yl]-2-(4-trifluoromethylphenyl)-3-(1H-1,2,4-triazol-1-yl)propan-2-ol obtained in Example 25 with 0.45 g of N-chlorosuccinimide (NCS), 0.35 g of the title compound was obtained (yield 38%).
m.p. 73-77°C (ethanol/white powder)
IR (cm ⁻¹, KBr): 1710, 1474, 1328, 1128, 961
NMR (ppm, CDCl₃ ): 4.36 (2H, s), 4.55 (1H, d), 4.92 (1H, d),6.38 (1H, s), 6.67 (1H, d), 6.91(1H, d), 7.1-7.7 (8H, m), 7.84 (1H, s)
MASS: 541 (M ⁺ )

### Example 31: 1-[3-[2-(4-Chlorophenyl)-trans-ethenyl]pyrrol-1-yl]-2-(2,4-difluorophenyl)-3-(1H-1,2,4-triazol-1-yl)propan-2-ol para-toluenesulfonate

0.20 g of 1-[3-[2-(4-Chlorophenyl)-trans-ethenyl]pyrrol-1-yl]-2-(2,4-difluorophenyl)-3-(1H-1,2,4-triazol-1-yl)propan-2-ol prepared in Example 1 was dissolved in 5 ml of dichloromethane. To the solution, 0.086 g of para-toluenesulfonic acid monohydrate was added to afford 0.18 g of the title salt (yield 65%).
m.p. 168-169°C (ethanol/colorless prisms)
IR (cm ⁻¹ , KBr): 3227, 3025, 1634, 1500, 1162, 1125, 1011, 813
NMR (ppm, DMSO-d₆ ): 2.29 (3H, s), 4.15-4.5 (2H, m), 4.47 (1H, d), 4.78 (1H, d), 6.01-7.7 (16H, m), 7.98 (1H, s), 8.55 (1H, s)

### Example 32: 1-[2-Chloro-3-[2-(4-fluorophenyl)-trans-ethenyl]pyrrol-1-yl]-2-(2,4-difluorophenyl)-3-(1H-1,2,4-triazol-1-yl)propan-2-ol m.p. 172-174°C (ethanol/white powder) IR (cm ⁻¹ , KBr): 3425, 1618, 1495, 1345, 1223, 1142, 1116, 967

Example 33: 1-[2-Chloro-3-[2-(4-(2,2,2-trifluoroethyl)oxyphenyl)-trans-ethenyl]pyrrol-1-yl]-2-(2,4-difluorophenyl)-3-(1H-1,2,4-triazol-1-yl)propan-2-ol
m.p. 146-148°C (ethanol/white powder)
IR (cm ⁻¹ , KBr): 3426, 1509, 1498, 1285, 1237, 1166, 966

## Claims

1. A triazole derivative represented by the following formula and a pharmacologically acceptable salt thereof: 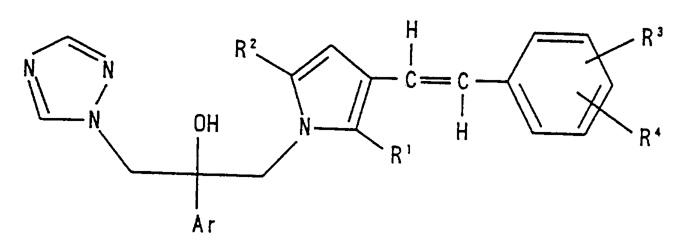 wherein Ar represents a phenyl group or a phenyl group substituted with one or two groups selected from the group consisting of halogen atoms and trifluoromethyl group; R¹ and R² independently represent a hydrogen atom, halogen atom, or a lower alkyl group; R³ and R⁴ independently represent a hydrogen atom, halogen atom, nitro group, a lower alkyl group, a lower halogeno-alkyl group, a lower alkoxy group, a lower halogeno-alkoxy group, non-substituted or substituted aryl group, non-substituted or substituted amino group, or a lower alkylthio group.

2. 1-[2-Chloro-3-[2-(4-chlorophenyl)-trans-ethenyl]pyrrol-1-yl]-2-(2,4-difluorophenyl)-3-(1H-1,2,4-triazol-1-yl)propan-2-ol according to claim 1.

3. 1-[3-[2-(4-Chlorophenyl)-trans-ethenyl]pyrrol-1-yl]-2-(2,4-difluorophenyl)-3-(1H-1,2,4-triazol-1-yl)propan-2-ol according to claim 1.

4. 1-[3-[2-(4-Trifluoromethylphenyl)-trans-ethenyl]pyrrol-1-yl]-2-(2,4-difluorophenyl)-3-(1H-1,2,4-triazol-1-yl)propan-2-ol according to claim 1.

5. A pharmaceutical composition comprising an effective amount of a triazole derivative or a salt thereof represented by the following formula together with a pharmaceutically acceptable carrier or coating: 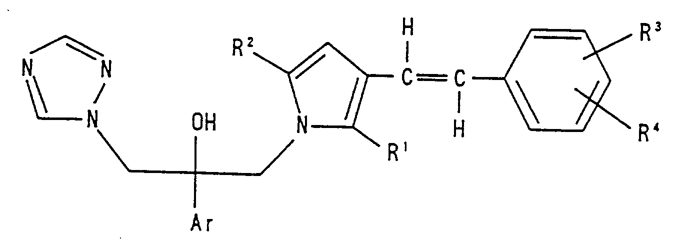 wherein Ar represents a phenyl group or a phenyl group substituted with one or two groups selected from the group consisting of halogen atoms and trifluoromethyl group; R¹ and R² independently represent a hydrogen atom, halogen atom, or a lower alkyl group; R³ and R⁴ independently represent a hydrogen atom, halogen atom, nitro group, a lower alkyl group, a lower halogeno-alkyl group, a lower alkoxy group, a lower halogeno-alkoxy group, non-substituted or substituted aryl group, non-substituted or substituted amino group, or a lower alkylthio group.

6. The pharmaceutical composition according to claim 5 useful for the treatment of a fungal infectious disease of an mammal host.

7. The pharmaceutical composition according to claim 5 useful for the treatment of mycosis profunda.

8. The pharmaceutical composition according to claim 5, which is administered orally to an mammal for the treatment of mycosis profunda.

9. The pharmaceutical composition according to claim 5, wherein the triazole derivative is selected from the group consisting of
1-[2-chloro-3-[2-(4-chlorophenyl)-trans-ethenyl]pyrrol-1-yl]-2-(2,4-difluorophenyl)-3-(1H-1,2,4-triazol-1-yl)propan-2-ol,
1-[3-[2-(4-chlorophenyl)-trans-ethenyl]pyrrol-1-yl]-2-(2,4-difluorophenyl)-3-(1H-1,2,4-triazol-1-yl)propan-2-ol,
1-[3-[2-(4-trifluoromethylphenyl)-trans-ethenyl]pyrrol-1-yl]-2-(2,4-difluorophenyl)-3-(1H-1,2,4-triazol-1-yl)propan-2-ol, and
a mixture thereof.

10. A method for treatment of a fungal infectious disease of a mammal host comprising a step of administering to the host an effective amount of a triazole derivative or a salt thereof represented by the following formula: 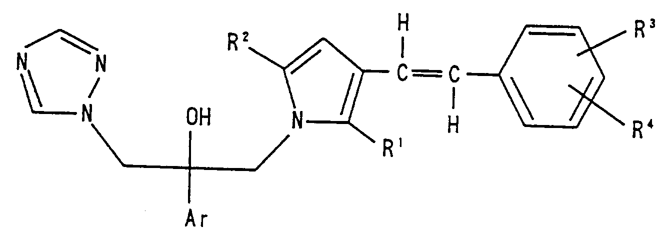 wherein Ar represents a phenyl group or a phenyl group substituted with one or two groups selected from the group consisting of halogen atoms and trifluoromethyl group; R¹ and R² independently represent a hydrogen atom, halogen atom, or a lower alkyl group; R³ and R⁴ independently represent a hydrogen atom, halogen atom, nitro group, a lower alkyl group, a lower halogeno-alkyl group, a lower alkoxy group, a lower halogeno-alkoxy group, non-substituted or substituted aryl group, non-substituted or substituted amino group, or a lower alkylthio group.

11. The method according to claim 10, wherein the fungal infectious disease is mycosis profunda.

12. The method according to claim 10, wherein the triazole derivative or the salt thereof is administered orally to the host.

13. The method according to claim 10, wherein the triazole derivative is selected from the group consisting of
1-[2-chloro-3-[2-(4-chlorophenyl)-trans-ethenyl]pyrrol-1-yl]-2-(2,4-difluorophenyl)-3-(1H-1,2,4-triazol-1-yl)propan-2-ol,
1-[3-[2-(4-chlorophenyl)-trans-ethenyl]pyrrol-1-yl]-2-(2,4-difluorophenyl)-3-(1H-1,2,4-triazol-1-yl)propan-2-ol,
1-[3-[2-(4-trifluoromethylphenyl)-trans-ethenyl]pyrrol-1-yl]-2-(2,4-difluorophenyl)-3-(1H-1,2,4-triazol-1-yl)propan-2-ol,
and a mixture thereof.

14. A compound represented by the following formula: 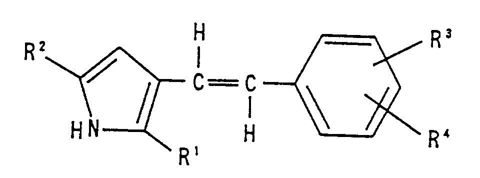 wherein R¹ and R² independently represent a hydrogen atom, a halogen atom, or a lower alkyl; R³ and R⁴ independently represent a hydrogen atom, a halogen atom, nitro group, a lower alkyl group, a lower halogeno-alkyl group, a lower alkoxy group, a lower halogeno-alkoxy group, a non-substituted or substituted aryl group, a non-substituted or substituted amino group, or a lower alkylthio group.
